(19) Europäisches Patentamt
European Patent Office
Office européen des brevets

(11) Numéro de publication: **0 115 713**
**B1**

# (12) FASCICULE DE BREVET EUROPEEN

(45) Date de publication du fascicule du brevet: **05.08.87**

(51) Int. Cl.⁴: **A 61 K 31/505**, C 07 D 239/42, C 07 D 239/46, C 07 D 403/12

(21) Numéro de dépôt: **83402143.8**

(22) Date de dépôt: **04.11.83**

(54) **(Pipérazinyl-1)-2 pyrimidines, leurs sels, procédé pour leur préparation et compositions pharmaceutiques en contenant.**

(30) Priorité: **09.11.82 FR 8218819**

(43) Date de publication de la demande: **15.08.84 Bulletin 84/33**

(45) Mention de la délivrance du brevet: **05.08.87 Bulletin 87/32**

(84) Etats contractants désignés: **AT BE CH DE FR GB IT LI LU NL SE**

(56) Documents cités:
**AT - B - 302 332**
**DE - B - 1 134 079**
**FR - A - 2 051 556**
**FR - M - 2 445**
**US - A - 2 979 508**
**US - A - 2 985 657**
**US - A - 4 247 549**

(73) Titulaire: **SANOFI, société anonyme, 40, Avenue George V, F-75008 Paris (FR)**

(72) Inventeur: **Carminati, Paolo, Via Pacini 24, Milan (IT)**
Inventeur: **Biziere, Kathleen, 6 Lotissement Rayons d'Oc, F-34170 Clapiers (FR)**
Inventeur: **Hallot, André, 83 rue de Juge, F-34980 Saint Gely du Fesc (FR)**

(74) Mandataire: **Gillard, Marie-Louise et al, Cabinet Beau de Loménie 55, Rue d'Amsterdam, F-75008 Paris (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

La présente invention concerne de nouvelles (pipérazinyl-1)-2 pyrimidines ayant une activité psychotrope dopaminergique, leurs sels, un procédé pour leur préparation ainsi que des compositions pharmaceutiques les renfermant en tant qu'ingrédiens actifs.

On a déjà décrit des dérivés de la pipérazine comme médicaments. A cet effet, on peut se référer notamment aux brevets AT-A-302 332, US-A-2 979 508 et US-A-2 985 657. Les composés décrits dans ces brevets appartiennent à la famille des butyrophénones dont les propriétés neuroleptiques sont bien connues.

On a maintenant trouvé des nouveaux dérivés de la pipérazine qui présente une activité dopaminergique.

Plus particulièrement, l'invention se réfère à des (pipérazinyl-1)-2 pyrimidines de formule:

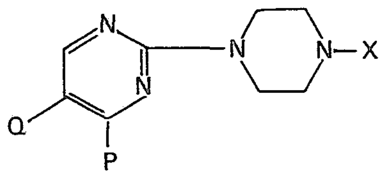

(I)

dans laquelle un des P et Q représente l'hydrogène, un groupe hydroxyle ou alkyl($C_{1-4}$)carbonyle et l'autre représente l'hydrogène; et X représente:

- un groupe CO-$R_1$, où $R_1$ est un alkyle en $C_{1-4}$
- un groupe

CO–CH–$R_2$,
|
NH–$R_3$

où $R_2$ est l'hydrogène, ou un groupe alkyle en $C_{1-4}$, phényle, p-hydroxyphényle, benzyle, p-hydroxybenzyle, hydroxyméthyle, 1-hydroxyéthyle ou 3-indolyméthyle et $R_3$ est de l'hydrogène, un alkyl($C_{1-4}$)carbonyle, un benzoyle, ou un groupe acyle dérivé d'un acide aminé choisi parmi: glycine, phénylglycine, alanine, valine, leucine, isoleucine, phénylalanine, tyrosine, sérine, thréonine ou tryptophane, ou encore un groupe acyle dérivé d'un dipeptide constitué à partir de deux des acides aminés mentionnés ci-dessus ou bien $R_2$ et $R_3$ pris ensemble forment un groupe éthylène;

- un groupe Alk-CODY, ou Alk représente un groupe alkylène à chaîne droite ou ramifiée contenant 1 à 4 atomes de carbone et Y est de l'hydrogène ou un alkyle en $C_{1-4}$
- un groupe Alk-$CH_2$-OZ, où Alk est tel que défini ci-desus et Z est l'hydrogène, ou un groupe alkyle en $C_{1-4}$, (alkoxy)-alkyle en $C_{1-4}$, alkyl($C_{1-4}$)carbonyle
- un groupe Alk-CO-W, où Alk est tel que défini ci-dessus et W est un groupe alkyle en $C_1$–$C_4$, ainsi qu'à leurs sels d'addition d'acides pharmaceutiquement acceptables.

Le terme «alkyle en $C_1$–$C_4$» tel qu'utilisé ici, désigne un radical dérivé d'un hydrocarbone aliphatique saturé contenant 1 à 4 atomes de carbone, tel que méthyle, éthyle, propyle, isopropyle, n-butyle et similaires.

Le terme «alkoxy en $C_1$–$C_4$» désigne le groupe hydroxyle substitué par un alkyle en $C_1$–$C_4$ tel que défini ci-dessus.

Le terme «alkylcarbonyle en $C_1$–$C_4$» désigne un radical

O
||
R–C– dans lequel R est un radical alkyle en $C_1$–$C_4$.

Les composés (I) selon la présente invention sont préparés selon le schéma réactionnel

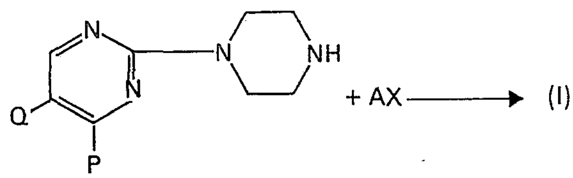


1 2

D'une façon générale, les composés (I) sont obtenus par substitution sur l'atome d'azote de la (pipérazinyl-1)-2 pyrimidine 1 par un dérive AX du substituant X à introduire. Le plus souvent A désigne un atome d'halogène et de préférence le chlore ou le brome.

Dans ce cas, on effectue la substitution au sein d'un solvant interte tel que le diméthylformamide ou le diméthylsulfoxyde en présence d'un agent alcalin minéral comme le carbonate de sodium, ou organique telle la triéthylamine à une température comprise entre 80°C et la température d'ébullition du solvant.

Dans le cas où X représente un groupe

CO–CH–$R_2$,
|
NH–$R_3$

on effectue la substitution avec l'acide

HOOC–CH–$R_2$
|
NH–$R_3$

et dans ce cas A représente un groupe activateur de la fonction carboxylique et notamment les groupes activateurs utilisés habituellement en chimie peptidique comme les esters activés de paranitrophényle ou N-hydroxysuccinimide ou encore les anhydrides mixtes avec le chloroformiate d'éthyle ou avec le chloroformiate d'isobutyle.

On peut également effectuer la substitution avec l'acide lui-même, en opérant en présence d'un carbodiimide et notamment le dicyclohexylcarbodiimide.

Pour que la substitution s'effectue de façon univoque, il est nécessaire dans le cas où $R_3$ représente l'hydrogène, de bloquer la fonction amine primaire par un groupe protecteur facile à éliminer ultérieurement, tel que les groupes benzyloxycarbonyle ou tertiobutyloxycarbonyle.

De plus, lorsque le substituant $R_2$ contient un ou plusieurs groupes susceptibles de réagir au cours de la réaction de substitution, et notamment des groupes hydroxyles; il est préférable de bloquer ces substituants par un groupe protecteur facile à éliminer ultérieurement. Dans le cas des groupes hydroxyles, on peut notamment utiliser un groupe éther benzylique.

Enfin, quand $R_3$ représente un groupe acyle dérivé d'un aminoacide ou d'un peptide, on peut, soit introduire directement comme indiqué précédemment l'ensemble du substituant

CO–CH–$R_2$,
      |
      NH–$R_3$

soit introduire selon le même procédé le substituant

CO–CH–$R_2$,
      |
      $NH_2$

puis procéder à l'élongation de la chaîne peptidique avec un ou deux autres aminoacides. Dans ce cas les fonctions $NH_2$ et OH de $R_3$ qui seraient éventuellement présentes doivent être bloquées sélectivement ainsi qu'il a été indiqué ci-dessus.

Les composés de formule I ci-dessus ainsi que leurs sels pharmaceutiquement acceptables possèdent une très bonne activité psychotrope avec un mécanisme d'action dopaminergique qui leur permet d'être utilisés comme médicaments dans des compositions pharmaceutiques pour le traitement des affections psychiques, neurologiques et neuromusculaires des mammifères, y compris l'être humain.

L'activité dopaminomimétique des produits de l'invention a été étudiée sur les récepteurs dopaminergiques striataux de la souris selon la technique décrite par P. PROTAIS et J. COSTENTIN Journal de Pharmacologie (Paris), 7 251–255, (1976). La lésion unilatérale des neurones dopaminergiques nigrostriataux induit une hypersensibilité des récepteurs de la dopamine au niveau du striatum. L'asymétrie qui en résulte est révélée par des rotations de l'animal dans le sens contralatéral aux récepteurs les plus intensément stimulés. Après administration des produits à étudier (0,1 mg/kg de poids corporel par voie orale), on compte pendant une période de 2 minutes le nombre de tours effectués par l'animal. On exprime les résultats sous forme de pourcentage des variations par rapport aux témoins n'ayant pas reçu de produits.

Les comptages sont effectués 3 heures après l'administration du produit et répétés après 6 heures. Dans ces conditions, les résultats suivants ont été obtenus:

| | | |
|---|---|---|
| SR 41697 A | 3 heures: –86% | 6 heures: –105% |
| SR 41902 | 3 heures: –71% | 6 heures: –71% |

Dans les compositions pharmaceutiques de la présente invention pour l'administration orale, sublinguale, sous-cutanée, intramusculaire, intraveineuse, transdermique ou rectale, les ingrédients actifs de formule I ci-dessus peuvent être administrés sous formes unitaires d'administration, en mélange avec des supports pharmaceutiques classiques, aux animaux et aux êtres humains pour le traitement des troubles de l'humeur ou du comportement, notamment dans le cas de psychoses, dépression ainsi que des états anxieux et des insomnies. Parmi les formes unitaires d'administration appropriées, il y a les formes par voie orale telles que les comprimés, les gélules, les poudres, les granules et les solutions ou suspensions orales et les formes d'administration parentérale utiles pour une administration sous-cutanée, intramusculaire ou intraveineuse.

Afin d'obtenir l'effet psychotrope désiré, la dose de principe actif peut varier entre 0,1 et 100 mg par kg de poids du corps et par jour. Chaque dose unitaire peut contenir de 1 à 300 mg d'ingrédient actif en combinaison avec un support pharmaceutique. Cette dose unitaire peut être administrée 1 à 4 fois par jour pour le traitement des troubles de l'humeur ou du comportement.

Les exemples suivants nullement limitatifs permettent de mieux comprendre la portée de l'invention.

Exemple 1

(Propionyl-4 pipérazinyl-1)-2 pyrimidine. (SR 41682)

(I) P = Q = H; X = $-COCH_2CH_3$

On dissout 5,6 g de maléate acide de (pipérazinyl-1)-2 pyrimidine dans 80 ml de diméthylformamide, puis on ajoute 5,6 ml de triéthylamine et 3,8 g de chlorure de propionyle. On chauffe pendant 12 heures à 130°C puis on évapore le solvant à siccité sous vide. On reprend le résidu dans une solution diluée d'acide chlorhydrique et lave avec de l'éther. On alcalinise la phase aqueuse avec une solution de carbonate de sodium et on extrait avec de l'acétate d'éthyle. On sèche la solution sur sulfate de sodium et évapore le solvant à siccité.

On chromatographie le résidu sur gel de silice. En éluant avec de l'acétate d'éthyle, on obtient le produit attendu. On recristallise dans l'éther isopropylique pour obtenir des cristaux (2,6 g). F: 98–100°C.

Exemple 2

(Ethoxycarbonylméthyl-4 pipérazinyl-1)-2 pyrimidine chlorhydrate. (SR 41697 A)

(I) P = Q = H; X = $-CH_2COOC_2H_5$

On opère comme dans l'exemple 1 en remplaçant le chlorure de propionyle par une quantité équivalente de bromacétate d'éthyle.

Après chauffage à 140°C pendant 6 heures, on isole le produit attendu comme indiqué dans l'exemple 1. Après chromatographie, le produit reste huileux. On le transforme en chlorhydrate

en ajoutant à la solution de la base dans l'éther, un excès d'une solution de gaz chlorhydrique dans le même solvant.

On essore les cristaux et recristallise deux fois dans l'éthanol absolu. F: supérieure à 260°C.

Exemple 3

(Acétonyl-4 pipérazinyl-1)-2 pyrimidine, chlorhydrate (SR 41722 A)

(I) P = Q = H; X = $CH_2COCH_3$

A la solution de 5,6 g de maléate acide de (pipérazinyl-1)-2 pyrimidine dans 100 ml de diméthylsulfoxyde, on ajoute 10,6 g de carbonate de sodium et 1,3 ml de chloracétone, puis on chauffe à 110°C durant 4 heures.

On évapore le solvant à siccité sous vide et reprend le résidu dans le minimum d'eau salée et extrait avec de l'acétate d'éthyle. On sèche la solution et évapore le solvant sous vide. On chromatographie le résidu sur gel de silice. En éluant avec de l'acétate d'éthyle, on élimine une impureté, puis, en éluant avec le mélange acétate d'éthyle-méthanol 90–10 vol/vol, on obtient le produit attendu sous forme d'huile.

On fabrique le chlorhydrate dans l'éther puis recristallise deux fois dans l'éthanol absolu.

On obtient finalement un solide hygroscopique. F: supérieure à 260°C.

Exemple 4

[(L-prolyl-L-leucyl-glycyl)-4 pipérazinyl-1]-2 pyrimidine trifluoracétate (SR 41902 A)

(I) P = Q = H X = -Gly-Leu-Pro

a) Boc-Leu-Gly-OMe

A la solution de 5,02 g de chlorhydrate de l'ester méthylique de la glycine dans un mélange de 20 ml de dichlorométhane et 30 ml de diméthylformamide, refroidie à 0°C, on ajoute 5 ml de N-éthylmorpholine puis la solution de 9,5 g de Boc-Leucine sèche dans 50 ml de dichlorométhane. On ajoute ensuite 9,04 g de dicyclohexylcarbodiimide, 6 g d'hydroxy-l benzotriazole et 1,5 ml de N-éthylmorpholine. Après 45 minutes à 0°C, on agite le mélange pendant 5 heures à température ambiante. On filtre l'insoluble qu'on lave avec de l'acétate d'éthyle. On dilue le filtrat avec 700 ml d'acétate d'éthyle et on lave la solution organique 3 fois avec une solution aqueuse de sulfate et bisulfate de potassium, puis 3 fois avec une solution saturée de bicarbonate de sodium et enfin 3 fois avec une solution saturée de clorure de sodium.

La solution organique séchée sur sulfate de sodium est concentrée sous vide à 200 ml. On ajoute 150 ml d'hexane et laisse cristalliser. On essore et lave avec de l'hexane.

On obtient ainsi des cristaux (9,90 g)

$[\alpha]_D^{20}$ = −20,3° (c = 1 diméthylformamide).

b) Trifluoroacétate de H-Leu-Gly-OMe

On agite durant 35 minutes 5 g du peptide obtenu en a) dans le mélange de 20 ml de dichlorométhane et 20 ml d'acide trifluoracétique. On évapore à siccité sous vide puis on reprend le résidu dans 150 ml d'éther. On évapore à siccité. On obtient un produit huileux (7 g) utilisé tel quel pour l'étape suivante.

c) Boc-Pro-Leu-Gly-OMe

Au produit obtenu en b) (7 g) dissous dans 55 ml de dichlorométhane, on ajoute de la N-éthylmorpholine pour amener le pH vers 6. On ajoute ensuite 3,55 g de Boc-Proline, 3,70 g de dicyclohexylcarbodiimide, 2,60 g d'hydroxy-1 benzotriazole et à nouveau de la N-éthylmorpholine pour ramener le pH vers 6. On laisse 2 heures 30 à température ambiante puis on filtre l'insoluble qu'on lave avec de l'acétate d'éthyle. Le filtrat dilué avec 500 ml d'acétate d'éthyle est lavé comme indiqué dans le paragraphe a). On sèche la solution sur sulfate de sodium puis concentre à 100 ml sous vide. On ajoute 100 ml d'hexane et essore le précipité. Le solide dissous dans le chloroforme est chromatographié sur colonne de silice (100 g). En éluant par le mélange chloroforme-méthanol 97-3 (vol/vol) on obtient le produit attendu. Après évaporation du solvant, on reprend le produit dans l'acétate d'éthyle et précipite par addition d'éther et d'hexane. On obtient un solide (4,45 g)

F: 103–105°C $[\alpha]_D^{20}$ = −59° (c = 1 diméthylformamide)

d) Boc-Pro-Leu-Gly-OH

A la solution de 4,8 g du produit obtenu ci-dessus dans 10 ml de méthanol et 10 ml de dioxanne, on ajoute 6 ml de solution de soude 1N. Après 15 minutes, on ajoute à nouveau 6 ml de solution de soude 1N et laisse 45 minutes. On rajoute 2 ml de la même solution de soude et laisse 2 heures. On ajoute 50 ml d'eau et 100 ml d'acétate d'éthyle et acidifie à pH = 2 avec une solution saturée de bisulfate de potassium. On sépare la phase organique et réextrait la phase aqueuse avec 500 ml d'acétate d'éthyle. On réunit les extraits organiques qu'on lave avec une solution de sulfate-bisulfate de potassium puis avec une solution saturée de chlorure de sodium.

On sèche la solution sur sulfate de sodium et concentre à siccité sous vide. On redissout le résidu dans 50 ml d'acétate d'éthyle et ajoute 150 ml d'hexane. On refroidit une heure au réfrigérateur et essore les cristaux formés qu'on lave avec de l'hexane.

Poids: 4,2 g F: 95–96°C $[\alpha]_D^{20}$ = −81° (c = 1 méthanol)

e) (Boc-Pro-Leu-Gly)-4 pipérazinyl-1]-2 pyrimidine

A la solution de 1,6 g de (pipérazinyl-1)-2 pyrimidine dans 50 ml de dichlorométhane, on ajoute 3,75 g du tripeptide obtenu en d) puis 2,20 g de dicyclohexylcarbodiimide, 1,50 g d'hydroxy-1 benzotriazole et 1,2 ml de N-éthylmorpholine. On laisse réagir durant 4 heures et filtre le précipité qu'on lave 3 fois avec 30 ml d'acétate d'éthyle. Le filtrat est dilué par 600 ml d'acétate d'éthyle et lavé 2 fois avec une solution de soude 0,5 N puis 4 fois avec une solution saturée de chlorure de sodium. On sèche sur sulfate de sodium et concentre sous vide. Le résidu est dissous dans 20 ml d'acétate d'éthyle et 5 ml de chloroforme.

On ajoute 150 ml d'éther et laisse 30 minutes. On essore les cristaux et lave avec de l'éther. On obtient ainsi 4,15 g du produit attendu
$[\alpha]_D^{20}$ = –78,3° (c = 1 méthanol).

f) SR 41902 A

On agite 4,08 g du produit obtenu ci-dessus avec 15 ml de dichlorométhane, 1,5 ml d'anisole et 18 ml d'acide trifluoracétique pendant 35 minutes. On concentre sous vide jusqu'à un volume de 7 à 8 ml et verse dans 150 ml d'éther. On obtient une gomme qu'on décante et lave par décantation avec 20 ml d'éther. On évapore sous vide et redissout le résidu dans 150 ml d'eau et ajoute de la résine Amberlite® IR 45 sous forme $OH^-$ jusqu'à pH de 8 environ. On filtre la résine qu'on lave 2 fois avec 50 ml d'eau. On concentre la phase aqueuse jusqu'à 30 ml puis on lyophilise le résidu. On obtient un solide blanc
(2,9 g) $[\alpha]_D^{20}$ = –50° (c = 1 eau)

Analyse d'acides aminés: Pro=0,9, Gly=1,03, Leu=0,97. Chromatographie sur couche mince de silice. Rf.=0,3 (chloroforme, méthanol, acide acétique 80-17-13 vol/vol).

Exemple 5

On prépare des gélules à base d'un des composés des exemples 1 à 4, ayant la composition suivante:

| | |
|---|---|
| principe actif | 15 mg |
| lactose | 120 mg |
| stéarate de magnésium | 5 mg |

en mélangeant intimement des charges des ingrédients ci-dessus et en versant le mélange dans des gélules de gélatine dure.

Exemple 6

On prépare des comprimés à base d'un des composés des exemples 1 à 4, ayant la composition suivante:

| | |
|---|---|
| principe actif | 20 mg |
| lactose | 100 mg |
| cellulose microcristalline | 30 mg |
| amidon de maïs séché | 40 mg |
| stéarate de magnésium | 5 mg |

en broyant l'ingrédient actif jusqu'à une dimension particulaire de 0,4 mm, en le faisant passer à travers un tamis de 0,4 mm d'ouverture de maille, en mélangeant la matière broyée avec les autres constituants et en comprimant pour former les comprimés.

De la même façon, on prépare des comprimés contenant 40 mg d'ingrédient actif.

Exemple 7

En opérant comme décrit dans l'exemple 6 ci-dessus, on prépare des comprimés ayant la composition suivante:

| | |
|---|---|
| principe actif | 50 mg |
| lactose | 95 mg |
| amidon de maïs | 100 mg |
| talc | 4,5 mg |
| stéarate de magnésium | 0,5 mg |

Exemple 8

On prépare des suppositoires ayant la composition suivante:

| | |
|---|---|
| principe actif | 50 mg |
| lactose | 250 mg |
| Witepsol® W 45 q.s.p. | 1,7 mg |

On mélange la substance active avec le lactose et on met le mélange uniformément en suspension dans la masse pour suppositoires fondue. On verse la suspension dans des moules refroidis pour former des suppositoires d'un poids de 1,7 g.

**Revendications Pour les états contractants: BE, CH, DE, FR, GB, IT, LI, LU, NL, SE**

1. (Pipérazinyl-1)-2 pyrimidines de formule

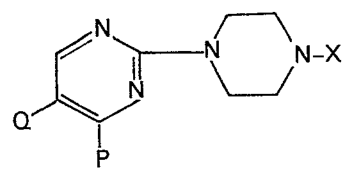

(I)

dans laquelle un des radicaux P et Q représente l'hydrogène, un groupe hydroxyle ou alkylcarbonyle dont le groupe alkyle est en $C_1$–$C_4$ et l'autre représente l'hydrogène, et X représente
- un groupe CO–$R_1$, où $R_1$ est un alkyle en $C_1$–$C_4$
- un groupe

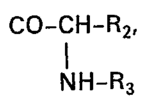

où $R_2$ est de l'hydrogène, ou un groupe alkyle en $C_1$–$C_4$, phényle, p-hydroxyphényle, benzyle, p-hydroxybenzyle, hydroxyméthyle, l-hydroxyéthyle, ou 3-indolylméthyle et $R_3$ est de l'hydrogène, un alkylcarbonyle dont le groupe alkyle est en $C_1$-$C_4$, un benzoyle, ou un groupe acyle dérivé d'un acide amino choisi parmi: glycine, phénylglycine, alanine, valine, leucine, isoleucine, phénylalanine, tyrosine, sérine, thréonine ou tryptophane, ou encore un groupe acyle dérivé d'un dipeptide constitué à partir de deux des acides aminés mentionnés ci-dessus ou bien $R_2$ et $R_3$ pris ensemble forment un groupe éthylène;
- un groupe Alk-COOY, ou Alk représente un groupe alkylène à chaîne droite ou ramifiée contenant 1 à 4 atomes de carbone et Y est de l'hydrogène ou un alkyle en $C_1$–$C_4$;
- un groupe Alk-$CH_2$-OZ, où Alk est tel que défini ci-dessus et Z est de l'hydrogène, ou un groupe alkyle en $C_1$–$C_4$ (alkoxy en $C_1$–$C_4$) -alkyle en $C_1$–$C_4$, alkylcarbonyle dont le groupe alkyle est en $C_1$–$C_4$;
- un groupe Alk-CO-W, où Alk est tel que défini ci-dessus et W est un groupe alkyle en $C_1$–$C_4$;

ainsi que leur sels d'addition d'acides pharmaceutiquement acceptables.

2. Composé selon la revendication 1, caractérisé en ce qu'il est la (Propionyl-4 pipérazinyl-1)-2 pyrimidine.

3. Composé selon la revendication 1, caractérisé en ce qu'il est l'(Ethoxycarbonylméthyl-4 pipérazinyl-1)-2 pyrimidine.

4. Composé selon la revendication 1, caractérisé en ce qu'il est l'(Acétonyl-4 pipérazinyl-1)-2 Pyrimidine.

5. Composé selon la revendication 1, caractérisé en ce qu'il est la (L-propyl-L-leucyl-glycyl)-4 pipérazinyl-1 -2 pyrimidine.

6. Procédé de préparation des produits selon l'une quelconque des revendications 1 à 5, caractérisé en ce que l'on fait réagir un dérivé de la (pipérazinyl)-2 pyrimidine de formule

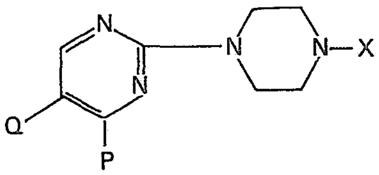

(I)

sur un composé de formule AX dans lequel A est choisi parmi un atome d'halogène et un groupe activateur de la fonction carboxylique du radical X.

7. Médicaments caractérisés en ce qu'ils contiennent au moins un produit selon l'une quelconque des revendications 1 à 5.

8. Médicaments selon la revendication 7, caractérisés en ce qu'ils contiennent entre 1 et 300 mg de produit selon l'une quelconque des revendications 1 à 5.

**Revendications pour l'état contractant: AT**

1. Procédé de préparation de (pipérazinyl-1) 2-pyrimidines de formule:

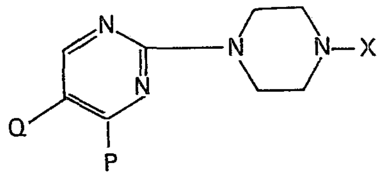

(I)

dans laquelle un des radicaux P et Q représente l'hydrogène, un groupe hydroxyle, ou alkyl($C_1$-$C_4$) carbonyle, et l'autre représente l'hydrogène, et X représente

- un groupe CO-$R_1$, ou $R_1$, est un alkyle en $C_1$-$C_4$
- un groupe

CO-CH-$R_2$,
|
NH-$R_3$

où $R_2$ est de l'hydrogène, ou un groupe alkyle en $C_1$-$C_4$, phényle, p-hydroxyphényle, benzyle, p-hydroxybenzyle, hydroxyméthyle, 1-hydroxyéthyle ou 3-indolylméthyle et $R_3$ est de l'hydrogène, un alkyle ($C_1$-$C_4$) carbonyle, un benzoyle, ou un groupe axyle dérivé d'un acide amino choisi parmi: glycine, phénylglycine, alanine, valine, leucine, isoleucine, phénylalanine, tyrosine, sérine, thréonine ou tryptophane, ou encore un groupe acyle dérivé d'un dipeptide constitué à partir de deux des acides aminés mentionnés ci-dessus ou bien $R_2$ et $R_3$ pris ensemble forment un groupe éthylène;

- un groupe Alk-COOY, où Alk représente un groupe alkylène à chaîne droite ou ramifiée contenant 1 à 4 atomes de carbone et Y est l'hydrogène ou un alkyle en $C_1$-$C_4$;
- un groupe Alk-$CH_2$-OZ où Alk est tel que défini ci-dessus et Z est de l'hydrogène, ou un groupe alkyle en $C_1$-$C_4$ (alkoxy en $C_1$-$C_4$)-alkyle en $C_1$-$C_4$, alkyl ($C_1$-$C_4$) carbonyle;
- un groupe Alk-CO-W, où Alk est tel que défini ci-dessus et W est un groupe alkyle en $C_1$-$C_4$; ainsi que leurs sels d'addition d'acides pharmaceutiquement acceptables, caractérisé en ce que l'on fait réagir un dérivé de la (pipérazinyl)-2-pyrimidine de formule:

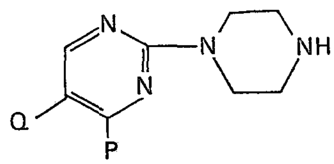

sur un composé de formule AX dans lequel A est choisi parmi un atome d'halogène, et un groupe activateur de la fonction carboxylique du radical X.

2. Procédé selon la revendication 1, caractérisé en ce que l'on fait réagir un dérivé de la (pipérazinyl-1)-2 pyrimidine de formule I dans laquelle P = Q = H avec un composé AX dans laquelle X est choisi parmi les radicaux ci-après: $-COCH_2-CH_3$, $-CH_2COOC_2H_5$, $-CH_2COCH_3$ et -Gly-Leu-Pro.

**Patentansprüche für die Vertragsstaaten: BE, CH, DE, FR, GB, IT, LI, LU, NL, SE**

1. 2-(1-Piperazinyl)-pyrimidine der Formel

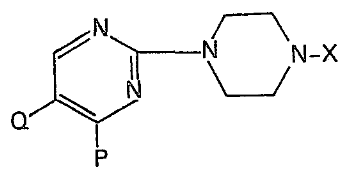

(I)

worin eines der Radikale P und Q Wasserstoff, eine Hydroxyl- oder Alkylcarbonylgruppe, deren Alkylgruppe $C_1$-$C_4$-Alkyl ist, darstellt und das andere für Wasserstoff steht, und X

- eine Gruppe CO-$R_1$, worin $R_1$ $C_1$-$C_4$-Alkyl ist
- eine Gruppe

CO-CH-$R_2$,
|
NH-$R_3$

worin $R_2$ Wasserstoff oder eine $C_1$-$C_4$-Alkylgruppe, Phenyl, p-Hydroxyphenyl, Benzyl, p-Hydroxybenzyl, Hydroxymethyl, 1-Hydroxäthyl oder 3-Indolylmethyl, und $R_3$ Wasserstoff, Alkylcarbonyl, dessen Alkylgruppe in $C_1$-$C_4$-Alkyl ist, Benzoyl oder eine Acylgruppe, die von einer Aminosäure, ausgewählt aus Glycin, Phenylglycin, Alanin, Valin, Leucin, Isoleucin, Phenylalanin, Tyrosin, Serin, Threonin oder Tryptophan, abstammt, oder auch eine Acylgruppe, die von einem Dipeptid, gebildet aus zwei der obengenannten Aminosäuren, abstammt, darstellen oder aber $R_2$ und $R_3$ zusammen eine Äthylengruppe bilden;

- eine Gruppe Alk-COOY, worin Alk für eine Alkylengruppe mit gerader oder verzweigter Kette, die 1 bis 4 Kohlenstoffatome enthält, steht und Y Wasserstoff oder $C_1$-$C_4$-Alkyl bedeutet;
- eine Gruppe Alk-$CH_2$-OZ, worin Alk obige Bedeutung hat und Z für Wasserstoff oder eine $C_1$-$C_4$-Alkyl-, ($C_1$-$C_4$-Alkoxy-)-$C_1$-$C_4$-Alkyl-, Alkylcarbonylgruppe, deren Alkylgruppe $C_1$-$C_4$-Alkyl ist, steht;
- eine Gruppe Alk-CO-W, worin Alk obige Bedeutung hat und W eine $C_1$-$C_4$-Alkylgruppe ist;

bedeutet, sowie ihre pharmazeutisch akzeptablen Säureadditionssalze.

2. Verbindung nach Anspruch 1, dadurch gekennzeichnet, dass sie 2-(4-Propionyl-1-piperazinyl)-pyrimidin ist.

3. Verbindung nach Anspruch 1, dadurch gekennzeichnet, dass sie 2-(4-Äthoxycarbonylmethyl-1-piperazinyl)-pyrimidin ist.

4. Verbindung nach Anspruch 1, dadurch gekennzeichnet, dass sie 2-(4-Acetonyl-1-piperazinyl)-pyrimidin ist.

5. Verbindung nach Anspruch 1, dadurch gekennzeichnet, dass sie 2-/4-(L-Propyl-L-leucylglycyl)-1-piperazinyl/pyrimidin ist.

6. Verfahren zur Herstellung der Produkte nach einem der Ansprüche 1 bis 5, dadurch gekennzeichnet, dass man ein Derivat von 2-(Piperazinyl)-pyrimidin der Formel

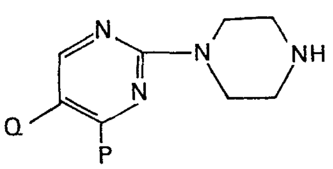

mit einer Verbindung der Formel AX, worin A ausgewählt ist aus einem Halogenatom und einer Aktivatorgruppe der Carboxylfunktion des Radikals X, reagieren lässt.

7. Medikamente, dadurch gekennzeichnet, dass sie mindestens ein Produkt nach einem der Ansprüche 1 bis 5 enthalten.

8. Medikamente nach Anspruch 7, dadurch gekennzeichnet, dass sie zwischen 1 und 300 mg des Produkts nach einem der Ansprüche 1 bis 5 enthalten.

**Patentansprüche für den Vertragsstaat: AT**

1. Verfahren zur Herstellung von 2-(1-Piperazinyl)-pyrimidinen der Formel

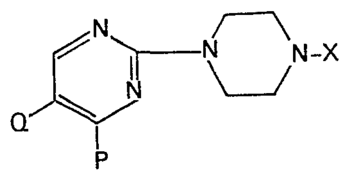

(I)

worin eines der Radikale P und Q Wasserstoff, eine Hydroxyl- oder $C_1$-$C_4$-Alkylcarbonylgruppe darstellt und das andere für Wasserstoff steht, und X

- eine Gruppe CO-$R_1$, worin $R_1$ $C_1$-$C_4$-Alkyl ist
- eine Gruppe

CO-CH-$R_2$,
|
NH-$R_3$

worin $R_2$ Wasserstoff oder eine $C_1$-$C_4$-Alkylgruppe, Phenyl, p-Hydroxyphenyl, Benzyl, p-Hydroxybenzyl, Hydroxymethyl, 1-Hydroxäthyl oder 3-Indolylmethyl, und $R_3$ Wasserstoff, $C_1$-$C_4$-Alkylcarbonyl, Benzoyl oder eine Acylgruppe, die von einer Aminosäure, ausgewählt aus Gylcin, Phenylglycin, Alanin, Valin, Leucin, Isoleucin, Phenylalanin, Tyrosin, Serin, Threonin oder Tryptophan, abstammt, oder auch eine Acylgruppe, die von einem Dipeptid, gebildet aus zwei der obengenannten Aminosäuren, abstammt, darstellen oder aber $R_2$ und $R_3$ zusammen eine Äthylengruppe bilden;

- eine Gruppe Alk-COOY, worin Alk für eine Alkylengruppe mit gerader oder verzweigter Kette, die 1 bis 4 Kohlenstoffatome enthält, steht und Y Wasserstoff oder $C_1$-$C_4$-Alkyl bedeutet;
- eine Gruppe Alk-$CH_2$-OZ, worin Alk obige Bedeutung hat und Z für Wasserstoff oder eine $C_1$-$C_4$-Alkyl-, ($C_1$-$C_4$-Alkoxy-)-$C_1$-$C_4$-Alkyl-, $C_1$-$C_4$-Alkylcarbonylgruppe steht;
- eine Gruppe Alk-CO-W, worin Alk obige Bedeutung hat und W eine $C_1$-$C_4$-Alkylgruppe ist;

deutet, sowie deren pharmazeutisch akzeptablen Säureadditionssalzen, dadurch gekennzeichnet, dass man ein Derivat von 2-(Piperazinyl)-pyrimidin der Formel

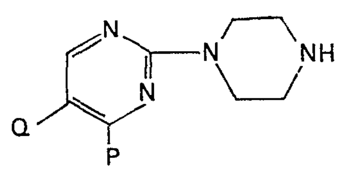

mit einer Verbindung der Formel AX, worin A ausgewählt ist aus einem Halogenatom und einer Aktivatorgruppe der Carboxylfunktion des Radikals X, reagieren lässt.

2. Verfahren nach Anspruch 1, dadurch gekennzeichnet, dass man ein Derivat von 2-(1-Piperazinyl)-pyrimidin der Formel I, worin P = Q = H, mit einer Verbindung AX, worin X ausgewählt ist aus den folgenden Gruppen: -$COCH_2$-$CH_3$, -$CH_2COOC_2H_5$, -$CH_2COCH_3$ und -Gly-Leu-Pro, reagieren lässt.

**Claims for the contracting states: BE, CH, DE, FR, GB, IT, LI, LU, NL, SE**

1. 2-(1-piperazinyl)pyrimidines of formula:

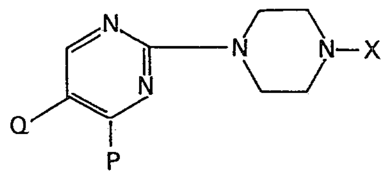

(I)

in which one of radicals P and Q represents hydrogen, a hydroxyl group, or alkylcarbonyl group, the alkyl group of which is in $C_1$–$C_4$ and the other represents hydrogen; and X represents:
- a group CO–$R_1$ where $R_1$ is an alkyl in $C_1$–$C_4$,
- a group

CO–CH–$R_2$
|
NH–$R_3$

where $R_2$ is hydrogen, or an alkyl group in $C_1$–$C_4$, phenyl, p-hydroxyphenyl, benzyl, p-hyroxybenzyl, hydroxymethyl, l-hydroxyethyl or 3-indolylmethyl group and $R_3$ is hydrogen, an alkylcarbonyl, the alkyl group of which is in $C_1$–$C_4$, a benzoyl or an acyl group derived from an amino acid selected from: glycine, phenylglycine, alanine, valine, leucine, isoleucine, phenylalanine, tyrosine, serine, threonine or tryptophane, or an acyl group derived from a dipeptide constituted from two of the amino acids mentioned above or $R_2$ and $R_3$ taken together form an ethylene group;
- a group Alk-COOY, where Alk represents an alkylene group with straight or branched chain containing 1 to 4 carbon atoms and Y is hydrogen or an alkyl in $C_1$–$C_4$;
- a group Alk-$CH_2$-OZ, where Alk is as defined hereinabove and Z is hydrogen, or an alkyl group in $C_1$–$C_4$, (alkoxy in $C_1$–$C_4$)-alkyl in $C_1$–$C_4$, alkylcarbonyl, the alkyl group of which is in $C_1$–$C_4$,
- a group Alk-CO-W, where Alk is as defined hereinabove and W is an alkyl group in $C_1$–$C_4$; as well as the pharmaceutically acceptable acid addition salts thereof.

2. Compound according to claim 1, characterized in that it is 2-(4-propionyl-1-piperazinyl)pyrimidine.

3. Compound according to claim 1, characterized in that it is 2-(4-ethoxycarbonylmethyl-1-piperazinyl)-pyrimidine.

4. Compound according to claim 1, characterized in that it is 2-(4-acetonyl-1-piperazinyl) pyrimidine.

5. Compound according to claim 1, characterized in that it is 2-[4-(L-propyl-L-leucyl-glycyl)-1-piperazinyl]-pyrimidine.

6. A process for preparing the products according to any one of claims 1 to 5, characterized in that it consists in reacting a derivative of the 2-(piperazinyl)pyrimidine of formula

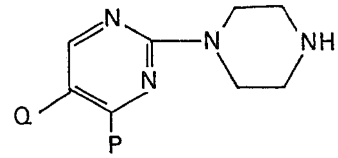

on a compound of formula AX in which A is selected from an atom of halogen and an activator group of the carboxylic function of the radical X.

7. Drugs, characterized in that they contain at least one product according to any one of claims 1 to 5.

8. Drugs according to claim 7, characterized in that they contain between 1 and 300 mg of the product according to any one of claims 1 to 5.

**Claims for the contracting state: AT**

1. Process for preparing the 2-(1-piperazinyl)pyrimidines of formula:

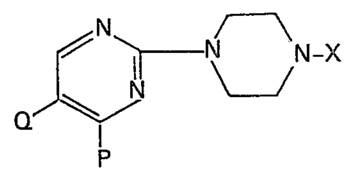

(I)

in which one of radicals P and Q represents hydrogen, a hydroxyl group, or alkyl ($C_1$–$C_4$)carbonyl group and the other represents hydrogen; and X represents:
- a group CO–$R_1$ where $R_1$ is an alkyl in $C_1$–$C_4$
- a group

CO–CH–$R_2$
|
NH–$R_3$

where $R_2$ is hydrogen, or an alkyl in $C_1$–$C_4$, phenyl, p-hydroxyphenyl, benzyl, p-hydroxybenzyl, hydroxymethyl, l-hydroxyethyl or 3-indolylmethyl group and $R_3$ is hydrogen, an alkyl($C_1$–$C_4$)carbonyl, a benzoyl or an acyl group derived from an amino acid selected from: glycine, phenylglycine, alanine, valine, leucine, isoleucine, phenylalanine, tyrosine, serine, threonine or tryptophane, or an acyl group derived from a dipeptide constituted from two of the amino acids mentioned above or $R_2$ and $R_3$ taken together form an ethylene group;
- a group Alk-COOY, where Alk represents an alkylene group with straight or branched chain containing 1 to 4 carbon atoms and Y is hydrogen or alkyl in $C_1$–$C_4$;
- a group Alk-$CH_2$-OZ, where Alk is as defined hereinabove and Z is hydrogen, or an alkyl group in $C_1$–$C_4$, (alkoxy in $C_1$–$C_4$)-alkyl in $C_1$–$C_4$, alkyl($C_1$–$C_4$) carbonyl group;
- a group Alk-CO-W where Alk is as defined hereinabove and W is an alkyl group in $C_1$–$C_4$; as

well as the pharmaceutically acceptable acid addition salts thereof, characterized in that it consists in reacting a derivative of the 2-(piperazinyl) pyrimidine of formula

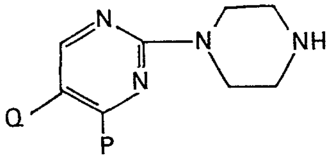

on a compound of formula AX in which A is selected from an atom of halogen and an activator group of the carboxylic function of the radical X.

2. Process according to claim 1, characterized in that a derivative of the 2-(1-piperazinyl)pyrimidine of formula I in which P = Q = H is reacted with a compound AX in which X is selected from among the following radicals:
$-COCH_2-CH_3$, $-CH_2COOC_2H_5$, $-CH_2COCH_3$ and -Gly-Leu-Pro.